# EUROPÄISCHE PATENTSCHRIFT

(11) **EP 1 430 854 B1**
(45) Veröffentlichungstag und Bekanntmachung des Hinweises auf die Patenterteilung: **23.02.2011**
(21) Anmeldenummer: 03090429.6
(22) Anmeldetag: 10.12.2003
(51) Int. Cl.: A61F 2/90

(54) **Stent**
Stent
Stent

(30) Priorität: 20.12.2002 DE 10261822
(43) Veröffentlichungstag der Anmeldung: 23.06.2004
(73) Patentinhaber: Biotronik GmbH & Co. KG, 12359 Berlin (DE)
(72) Erfinder: Lootz, Daniel, 18119 Warnemünde (DE); Koop, Karsten, 18057 Rostock (DE); Bakczewitz, Frank, 18055 Rostock (DE); Kiekbusch, Martin, 18437 Stralsund (DE)
(74) Vertreter: Lindner-Vogt, Karin L.

(56) Entgegenhaltungen:
- WO-A-99/38458
- WO-A-02/091958
- WO-A1-00/16718
- DE-U- 20 019 429
- US-A- 6 042 597
- US-A1- 2002 095 206

## Beschreibung

Die Erfindung betrik einen Stent mit einer Längsachse und eine Umfangswandung, die wenigstens über eine Teillänge des Stents von einer an zwei Stirnseiten offenen, gitterartigen Tragstruktur gebildet ist. Diese Tragstruktur umfasst genau einen derartig helixartig um die Längsachse umlaufenden Steg, dass sich eine helixförmige Primärform des umlaufenden Stegs mit mehr als einer Helixwindung ergibt. Der Steg weist neben dieser helixartigen Primärform eine zick-zack-artige oder mäanderförmige Sekundärform auf. Die Tragstruktur weist außerdem Verbindungsstege auf, die jeweils eine Helixwindung der helixartigen Primärform überbrücken.

Stents im Allgemeinen sind endovaskuläre Prothesen, die beispielsweise zur Behandlung von Stenosen verwendet werden. Stents sind außerdem bekannt für die Behandlung von Aneurismen.

Stents weisen grundsätzlich eine Tragstruktur auf, die geeignet ist, die Wand eines Gefäßes in geeigneter Weise abzustützen, um das Gefäß so zu weiten bzw. ein Aneurisma zu überbrücken. Stents werden dazu in einem komprimierten Zustand in das Gefäß eingeführt und dann an dem zu behandelnden Ort aufgeweitet und gegen die Gefäßwand gedrückt. Dieses Aufweiten kann beispielsweise mit Hilfe eines Ballonkatheders erfolgen. Alternativ sind auch selbstexpandierende Stents bekannt. Diese sind beispielsweise aus einem Memorymetall wie Nitinol hergestellt, welches bei erreichen einer bestimmten Sprungtemperatur seine Form ändert, nämlich im vorliegenden Fall von der komprimierten Form zur expandierten Form.

Die wünschenswerten Eigenschaften eines Stents sind vielfältiger Art und teilweise auf verschiedenste Art verwirklicht. Gewünscht ist eine möglichst einfache Expandierbarkeit des Stents bei gleichzeitig einfacher Positionierbarkeit. Bei ballonexpandierbaren Stents ist außerdem ein möglichst geringes elastisches Zurückfedern (Recoil) nach der Expansion gewünscht, um den Stent nicht all zu sehr über das gewünschte Maß hinaus expandieren zu müssen. Bei selbstexpandierenden Stents wie auch bei ballonexpandierenden Stents ist eine ausreichenden Tragkraft gewünscht. Im Zusammenhang mit medikamentenbeschichteten Stents ist außerdem eine möglichst gleichmäßige Flächenabdeckung der Gefäßwand gewünscht. Schließlich soll der Stent auch eine gewisse Biegsamkeit um seine Längsachse besitzen, um entsprechende Gefäßbewegungen mitzumachen. Dies soll allerdings nicht auf Kosten der Tragkraft des Stents geschehen. Der Stent soll außerdem eine möglichst gute Seitenastzugänglichkeit bieten. Weiterhin ist ein Anbringen von Stegen der Tragstruktur, ein sogenanntes Tishscaling in Regel unerwünscht. Die Liste der hier genannten wünschenswerten Eigenschaften ist bei weitem nicht abschließend.

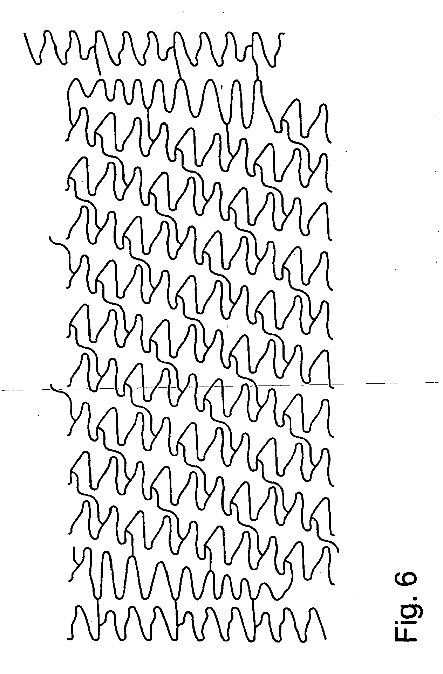

Unter den vielfältigen Versuchen, die wünschenswerten Eigenschaften eines Stents in Einklang miteinander zu bringen, findet sich auch der Stent gemäß US 5,925,061. Dieses US-Patent zeigt eine ganze Reihe unterschiedlich gestalteter Stents, unter anderem in den Figuren 6 bis 10 einen Stent mit einer helixartig um die Längsachse des Stents umlaufenden Tragstruktur.

Aus US-A-6042597 ist ein Stent bekannt, der die technischen Merkmale aus dem Oberbegriff aus Anspruch 1 beinhaltet.

Aus WO 02/091958 ist ein Stent bekannt, dessen Tragstruktur von einer Vielzahl von in Längsrichtung des Stents hintereinander angeordneten, in Umfangsrichtung umlaufenden Stegen gebildet ist, die dementsprechend jeweils eine ringförmige Primärform besitzen. Diese Stege weisen außerdem eine mäanderförmige Sekundärform auf und sind in Längsrichtung des Stents jeweils an drei Verbindungsstellen mit dem jeweils benachbarten ringförmigen Steg verbunden. Die Verbindungsstellen sind dabei so angeordnet, dass sie jeweils auf einer von drei helixartig um den Stent herum laufenden Verbindungslinien liegen.

Trotz der Vielzahl der bestehenden Stentkonstruktionen besteht nach wie vor das Bedürfnis, die Vielzahl der unterschiedlichen wünschenswerten Eigenschaften eines Stents für einen jeweiligen Anwendungsfall möglichst gut aufeinander abzustimmen.

Entsprechend liegt der vorliegenden Erfindung die Aufgabe zu Grunde, einen Stent der Eingangs genannten Art derart weiterzubilden, dass er dem Fachmann bezüglich der Verwirklichung seiner wünschenswerten Eigenschaften eine weitere Option bietet.

Erfindungsgemäß wird diese Aufgabe durch einen Stent der Eingangs genannten Art gelöst, bei dem die Verbindungsstege nicht an Wendeabschnitten der Sekundärform ansetzen, sondern an Teilstegen der helixförmigen Tragstruktur, die sich jeweils zwischen zwei Wendeabschnitten der Tragstruktur erstrecken und zwei benachbarte Wendeabschnitte miteinander verbinden.

Ein derartiger Stent unterscheidet sich in seinen Eigenschaften von den aus dem Stand der Technik bekannten Stents zum Beispiel durch sein Verhalten beim Expandieren, insbesondere hinsichtlich möglicher Längenveränderungen. Der Stent unterscheidet sich aber auch dadurch vom Stand der Technik, dass er unter Beibehaltung sonstiger wünschenswerter Eigenschaften eine vorteilhafte Flächenabdeckung bietet.

Der Stent ist vorzugsweise beispielsweise mit Hilfe eines Lasers aus einem rohrförmigen Ausgangswerkstück geschnitten.

Vorzugsweise umfasst das Material des Stents Metalle oder Legierungen wie Nitinol, Titan, Tantal oder Stahl, beispielsweise Edelstahl 316 L. Alternativ kommen auch korrodierbare Materialien, insbesondere korrodierbare Metalle wie Magnesium oder Eisen als Material für den Stent in Frage. Es kommen Kobaltbasislegierungen, Nickelbasislegierungen, Kupferbasislegierungen und Magnesiumlegierungen in Frage. Geeignete Titan-Tantal-Legierungen sind TiTa₃₀, geeignete Titan-Niob-Legierungen sind TiNb₃₀. Alternativ eignen sich auch ADI-Legierungen auf Palladium- und Silberbasis oder Algiloy. Auch Thermoplaste stellen grundsätzlich geeignete Materialien dar, insbesondere bioresorbierbare Thermoplasten.

Die Wanddicke des rohrförmigen Ausgangswerkstückes und damit die Dicke der durch Schneiden erzeugten Stege ist vorzugsweise über die gesamte Länge wie auch über den Umfang des Stents identisch. Alternativ ist die Dicke der Stege im Bereich der beiden Stirnenden des Stents über den Umfang des Stents gleichmäßig gegenüber der Stegdicke im mittleren Bereich des Stents verringert.

Der für die Beschreibung der Geometrie des Stents verwendete Begriff Erstreckungskomponente meint jeweils die möglichen Erstreckungskomponenten in Längsrichtung bzw. Umfangsrichtung des Stents. Ein Steg, der parallel zur Längsachse des Stents verläuft, hat lediglich eine Erstreckungskomponente in Längsrichtung des Stents. Ein Steg, der genau in Umfangsrichtung des Stents verläuft, hat lediglich eine Erstreckungskomponente in Umfangsrichtung und keine Erstreckungskomponente in Längsrichtung des Stents. Schräg zur Längsachse des Stents und zur Umfangrichtung des Stents verlaufende Stege haben Erstreckungskomponenten sowohl in Längsrichtung als auch in Umfangsrichtung.

Zwei mit einem Teilsteg verbundene und somit unmittelbar benachbarte, abgerundete Wendeabschnitte der Sekundärform begrenzen den jeweiligen, von diesen Wendeabschnitten eingeschlossenen Abschnitt der helixartigen Primärform in Längsrichtung des Stents.

In einer bevorzugten Ausführungsvariante sind die Teilstege, die zwei benachbarte Wendeabschnitte verbinden, nicht vollständig gestreckt, sondern weisen eine flache S-Form auf, so dass sich eine flach-S-förmige Tertiärform des umlaufenden Stegs ergibt.

Ebenso ist es bevorzugt, wenn auch die Verbindungsstege nicht vollständig gestreckt sind sondern eine flache S-Form aufweisen.

Die Verbindungsstege haben vorzugsweise eine Haupterstreckungskomponente in Längsrichtung des Stents. Dies bedeutet, dass die Erstreckungskomponente in Längsrichtung des Stents größer ist als die Erstreckungskomponente in Umfangsrichtung des Stents, so dass die Verbindungsstege vorzugsweise in einem flachen Winkel zur Längsachse des Stents verlaufen.

Vorzugsweise ist jeweils eine Helixwindung der helixartigen Primärform von mehreren Verbindungsstegen überbrückt. Diese Verbindungsstege sind über eine Helixwindung vorzugsweise soweit möglich gleichmäßig verteilt. Eine bevorzugte Anzahl von Verbindungsstegen pro Helixwindung ist zwei bis fünf.

Bei einer bevorzugten Tragstruktur, welche mehrere Helixwindungen aufweist, sind diese Helixwindungen durch Verbindungsstege vorzugsweise derart überbrückt, dass die Verbingundsstege benachbarter Helixwindungen in Umfangsrichtung des Stents zueinander versetzt sind.

Außerdem weist die Umfangswandung des Stents wenigstens eine an eine Stirnseite der Tragstruktur angrenzendende Endstruktur auf, die ein an seiner Stirnseite offenes Längsende der Umfangswandung bildet. Diese Endstruktur umfasst einen ringförmig entlang der Umfangsrichtung des Stents an dessen Stirnseite umlaufenden Tragabschnitt auf, von dem die Tragstruktur mit der helixartigen Primärform abzweigt. Der ringförmige Tragabschnitt wird vorzugsweise von einem umlaufenden, mäander- oder zick-zack-artiggewellten Steg gebildet. Vorzugsweise sind zwischen der ringförmigen Tragstruktur und der an sie angrenzenden erste Helixwindungen Verbindungsstege vorgesehen.

Die Erfindung soll nun anhand eines Ausführungsbeispiels mit Bezug auf die Figuren näher beschrieben werden. Die Figuren zeigen:
- Figur 1: ein erstes Beispiel eines Stents (nicht beanspruncht) mit einer von Stegen gebildeten, gitterartigen Umfangswandung;
- Figur 2: einen Querschnitt durch einen Steg;
- Figur 3: eine Abwicklung der Umfangswandung des Stents aus Figur 1;
- Figur 4: einen vergrößerten Ausschnitt der Abwicklung mit einem Detail der Tragstruktur;
- Figur 5: einen vergrößerten Ausschnitt der Abwicklung aus Figur 1 mit der Endstruktur des Stents;
- Figur 6: ein alternatives Beispiel eines Stents;
- Figur 7: eine Ausführung eines Stents;
- Figur 8: eine vergrößerte Darstellung eines Endabschnitts des Stents aus Figur 7;
- Figur 9: eine alternative Variante eines Stents in der Seitenansicht;
- Figur 10: eine vergrößerte Darstellung eines Endabschnitts des Stents aus Figur 9; und
- Figur 11: eine vergrößerte Darstellung eines mittleren Abschnitts des Stents aus den Figuren 7 bis 11.

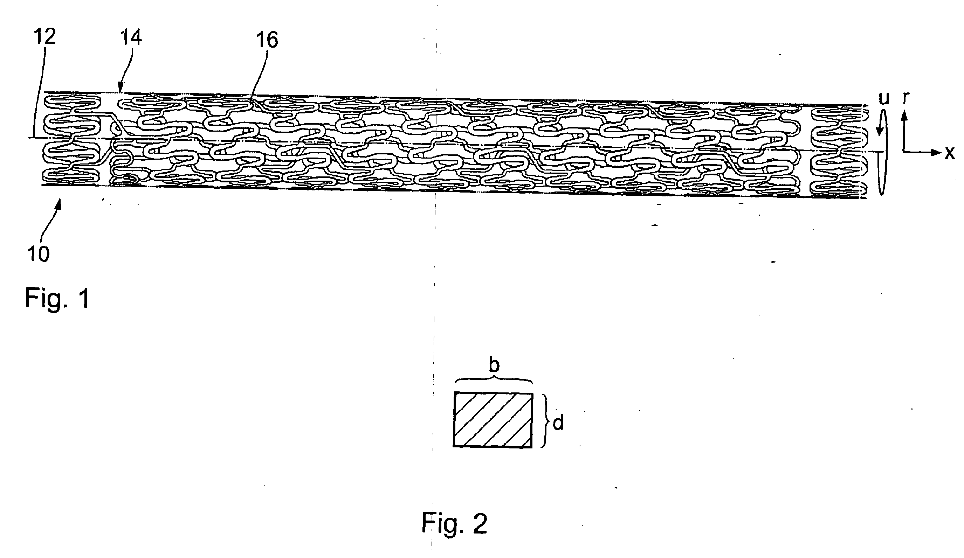

In Figur 1 ist ein Stent 10 dargestellt, der eine Längsachse 12 aufweist, und eine die Längsachse 12 umschließende Umfangswandung 14, die eine Tragstruktur für den Stent 10 bildet. Die Umfangswandung 14 und die von ihr gebildete Tragstruktur ist von Stegen 16 gebildet.

Zur Beschreibung der Geometrie der Umfangswandung 14 werden die in Figur 1 dargestellten Koordinaten verwendet, nämlich x als Längsrichtung des Stents 10, r als radial zur Längsrichtung x verlaufenden Koordinate und u als in Umfangsrichtung des Stents 10 verlaufende Koordinate.

Dem in Figur 2 dargestellten Querschnitt durch einen Steg 16 ist zu entnehmen, dass die Steggeometrie durch eine Stegdicke d und eine Stegbreite b beschrieben werden kann. Die Stegdicke d ist dabei die Ausdehnung eines Stegs in radialer Richtung r bezüglich des Stents 10, während die Stegbreite b die Ausdehnung eines Stegs in Richtung einer von der Umfangswandung 14 gebildeten Mantelfläche darstellt.

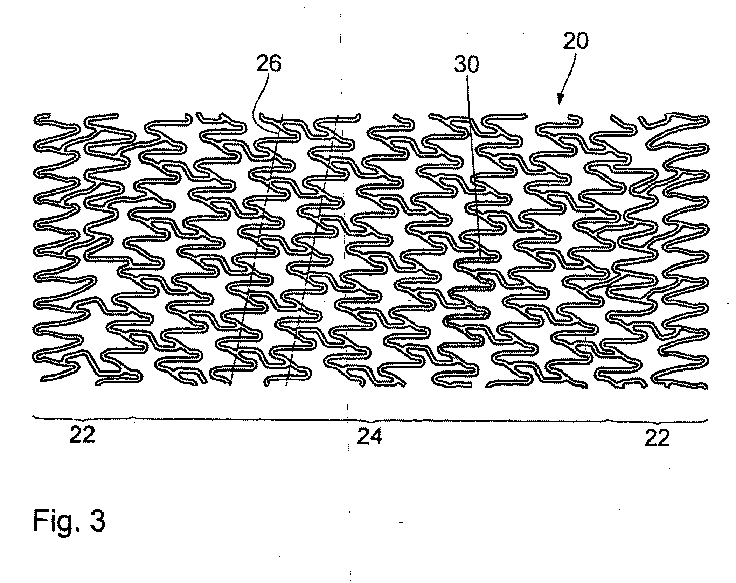

Figur 3 zeigt eine Abwicklung 20 der Umfangswandung 14. Es ist zu erkennen, dass die Umfangswandung 14 an beiden Längsenden Endstrukturen 22 aufweist, welche eine zentrale Tragstruktur 24 einschlieβen.

Sowohl die Endstrukturen 22 als auch die zentrale Tragstruktur 24 werden von Stegen 16 gebildet. Diese haben vorzugsweise den in Figur 2 abgebildeten rechteckigen Querschnitt, wobei die Stegdicke d mit Bezug auf Figur 3 einer Erstreckung senkrecht zur Zeichenebene entspricht und die Stegbreite die Erstreckung eines Steges 16 in der Zeichenebene bezeichnet.

Die von den Stegen 16 gebildete zentrale Tragstruktur 24 weist eine helixartige Primärform auf, die durch Linien 26 in Figur 3 angedeutet ist.

Daneben weisen die die zentrale Tragstruktur 24 bildenden Stege 16 eines zick-zack- oder mäanderförmige Sekundärform auf, wie sie in Figur 3 durch die gepunktete Linie 30 dargestellt ist.

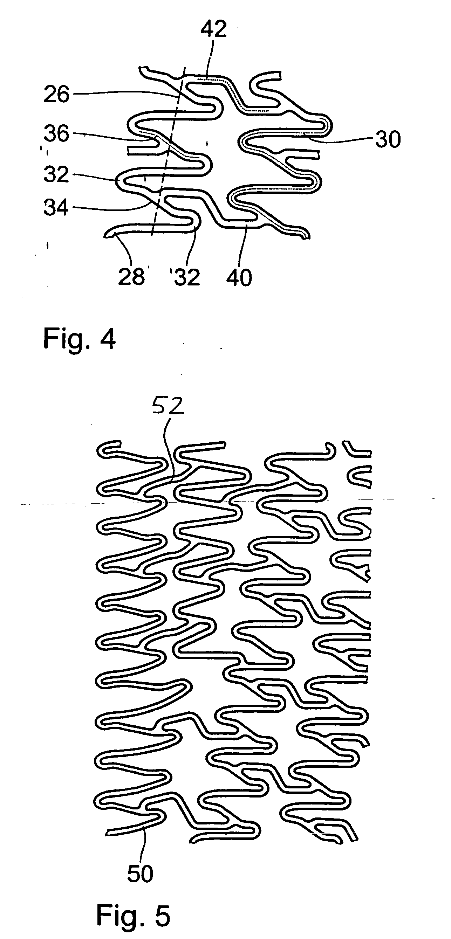

In Figur 4 ist ein Ausschnitt der zentralen Tragstruktur 24 vergrößert dargestellt. Wie der Figur 4 zu entnehmen ist, ist die zick-zack- oder mäanderförmige Sekundärform so ausgerichtet, dass die Stege 16 Wendepunkte 32 aufweisen, zwischen denen sich jeweils Teilstege 34 erstrecken, die eine Haupterstreckungskomponente in Längsrichtung des Stents aufweisen. Die Teilstege 34 haben darüber hinaus eine flach S-förmige Tertiärform, wie sie durch die gepunktete Linie 36 in Figur 4 dargestellt ist.

Wie außerdem den Figuren 3 und 4 zu entnehmen ist, weist die zentrale Tragstruktur 24 neben den in Mäandern helixförmig umlaufenden Stegen auch Verbindungsstege 40 auf, welche jeweils eine Helixwindung der helixartigen Primärstruktur überbrücken und welche an ihren beiden Längsenden an den Teilstegen 34 der helixförmigen Primärform zwischen jeweils zwei benachbarten Wendepunkten ansetzen. Auch die Verbindungsstege 40 besitzen eine flache S-Form, die in Figur 4 durch die gepunktete Linie 42 angedeutet ist. Die Verbindungsstege 40 haben, wie Figur 4 zu entnehmen ist, eine Haupterstreckungskomponente in Längsrichtung des Stents und verlaufen in einem flachen Winkel zur Längsrichtung des Stents.

Figur 3 ist zu entnehmen, dass zwei benachbarte Helixwindungen der zentralen Tragstruktur 24 durch 4 bis 5 Verbindungsstege 40 überbrückt sind. Die Verbindungsstege 40 sind dabei so angeordnet, dass die auf jeweils verschiedenen Seiten einer Helixwindung 26 angreifenden Verbindungsstege in Umfangsrichtung des Stents relativ zueinander versetzt sind.

Figur 5 zeigt eine Endstruktur des Stents in vergrößerter Darstellung. Zu erkennen ist, dass das Längsende der Umfangswandung von einem ringförmig umlaufenden, mäander- oder zick-zack-artig gefalteten Steg 50 gebildet wird. An diesen Stegring 50 ist über Verbindungsstege 52 die zentrale Tragstruktur 24 angeschlossen. Als Teil der Endstruktur 22 kann angesehen werden, dass die unmittelbar an den Ringsteg 50 angrenzende, äußere Helixwindung der zentralen Tragstruktur 24 dadurch ausläuft, dass die entsprechenden Teilsteg immer kürzer werden.

Figur 6 zeigt eine alternative Umfangswandung eines Stents in der Abwicklung, die sich von in den Figuren 1 bis 5 dargestellten Beispielen vor allem dadurch unterscheidet, dass die vorgesehenen Verbindungsstege anders angeordnet sind.

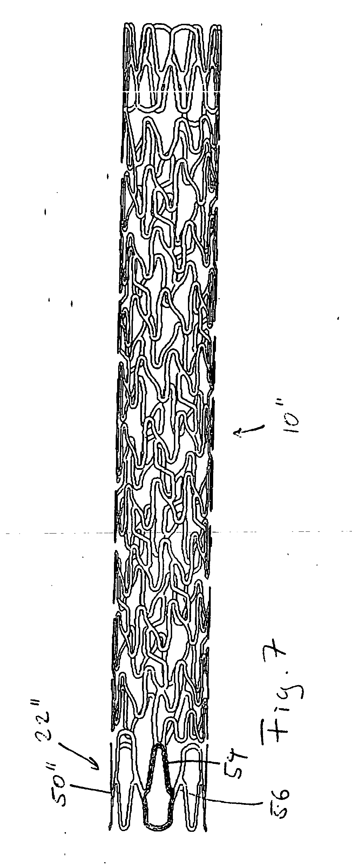

Der in Figur 7 in der Seitenansicht abgebildete Stent 10" unterscheidet sich von den vorangehenden Figuren dargestellten Stents durch die Gestaltung seiner Endstruktur 22". Diese ist von einem Stegring 50" gebildet, der von in Umfangsrichtung des Stents 10 benachbart angeordneten, geschlossenen Zellen gebildet ist, von denen eine geschlossene Zelle 54 in Figur 7 dunkel dargestellt ist. Benachbarte geschlossene Zellen 54 sind durch Umfangsverbindungsstege 56 miteinander verbunden. Eine derartige Endstruktur 22' besitzt vorteilhafte Eigenschaften bezüglich der Gleichmäßigkeit des Öffnungsverhaltens der zentralen Tragstruktur 24". Hinzu kommt ein günstiges Verhalten bezüglich der Längenänderung des Stents 10" beim Expandieren. Weiterhin wirkt sich ein Endabschnitt wie der Endschnitt 22" aufgrund seiner höheren radialen Stabilität günstig auf den Kollapsdruck des Stents aus, d.h. also auf die radial auf den Stent einwirkenden Kräfte, denen der Stent gewachsen ist. Schließlich wird auch das Haftenbleiben des Stents auf einem Ballonkatheter beim Einführen verbessert.

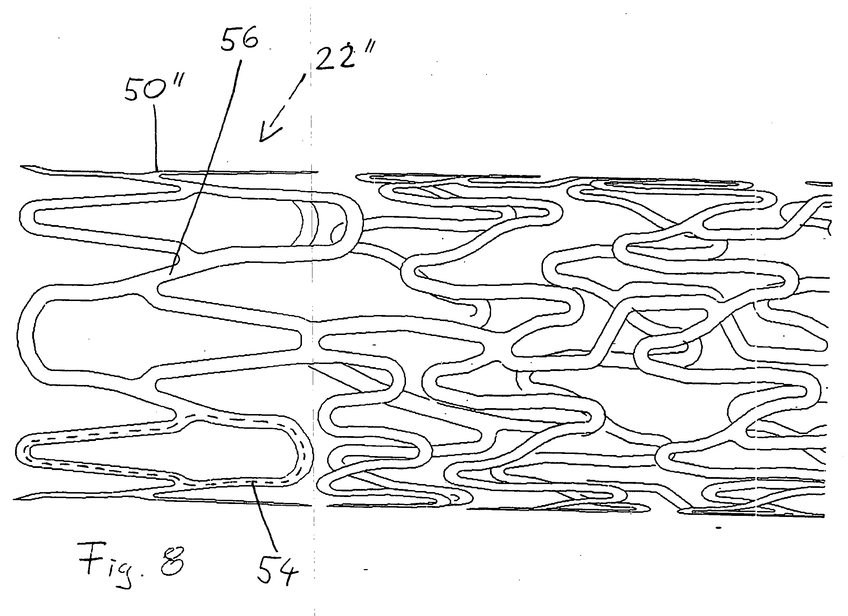

Figur 8 zeigt den Endabschnitt 22" aus Figur 7 in vergrößerter Darstellung.

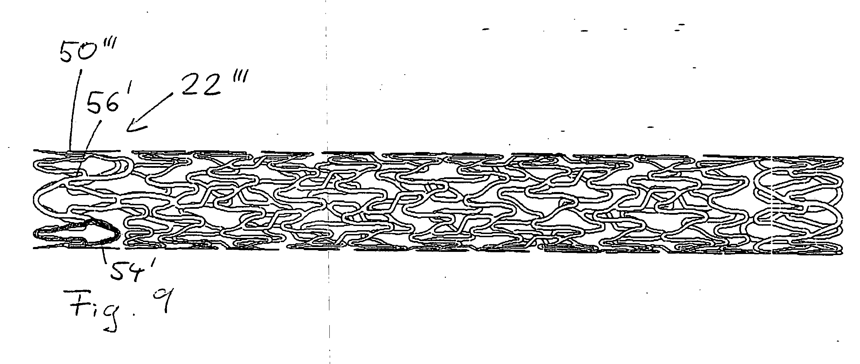

Die in Figur 9 dargestellte Variante unterscheidet sich von der in Figur 7 dargestellten Variante durch die genaue Form der geschlossenen Zellen 54'.

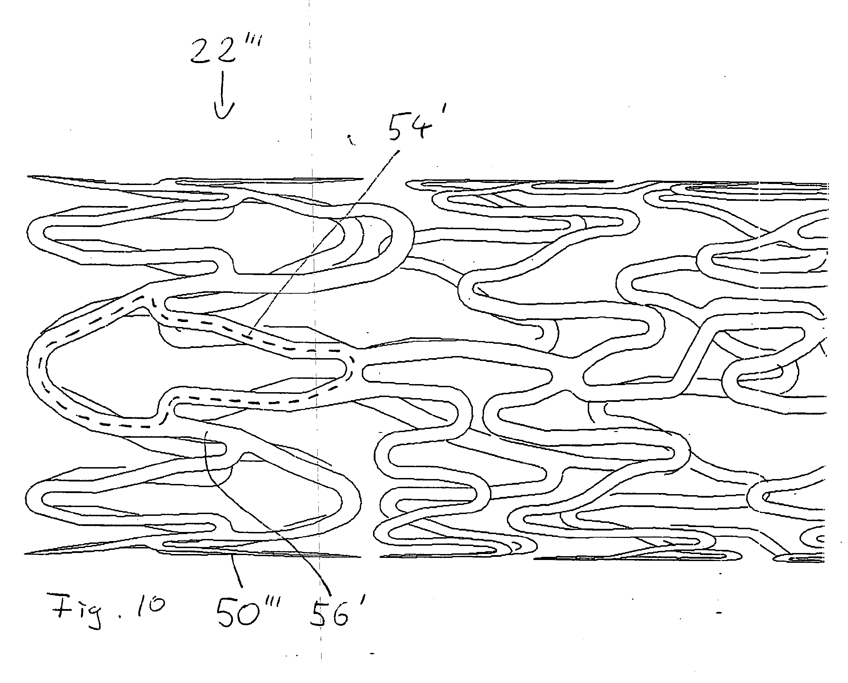

Figur 10 zeigt den Endabschnitt 22''' aus Figur 9 in vergrößerter Darstellung.

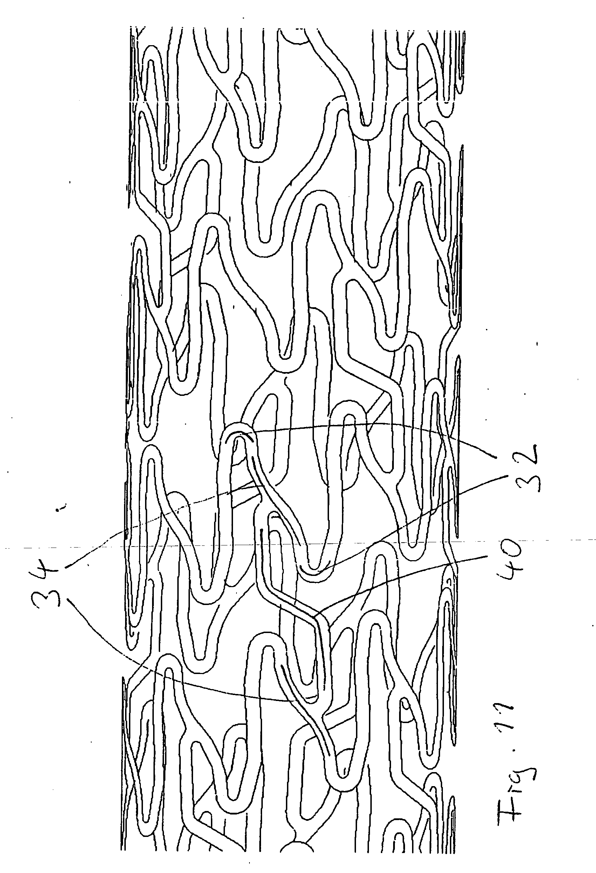

In Figur 11 ist schließlich eine zentrale Tragstruktur 22" in vergrößertem Ausschnitt dargestellt, wie sie den Stentvarianten aus den Figuren 7 bis 10 gemein ist.

Den Figuren nicht zu entnehmen ist, dass die Stege der Endstrukturen 22 und der zentralen Tragstruktur 14 unterschiedliche Stegdicken d aufweisen können.

Geeignete Materialien, aus denen die in den Figuren abgebildeten Tragstrukturen bestehen können, sind in der Beschreibungseinleitung angegeben. Die dargestellten Umfangswandungen können je nach Anwendungsfall Wirkstoffe tragen, die beispielsweise mit Hilfe einer auf die Umfangswandung aufgebrachten Polymermatrix an die Umfangswandung des Stents angebunden sind.

## Patentansprüche

1. Stent
mit einer Längsachse (12) und
mit einer Umfangswandung (14),
die wenigtstens über eine Teillänge des Stents (10) von einer an zwei Stirnseiten offenen, gitterartigen Tragstruktur (24) gebildet ist, die genau einen derart helixartig um die Längsachse umlaufenden Steg (16) umfasst, dass sich eine helixförmige Primärform (26) des umlaufenden Stegs mit mehr als einer Helixwindung ergibt
wobei der Steg (16) neben der helixartigen Primärform (26) eine zickzackartige oder mäanderförmige Sekundärform (30) mit Wendepunkten und zugehörigen Wendeabschnitten (32) besitzt, zwischen denen Teilstege (34) des umlaufenden Stegs mit einer Erstreckungskomponente in Längsrichtung des Stents verlaufen, und
wobei die Tragstruktur weiterhin Verbindungsstege (40) umfasst, welche jeweils eine Helixwindung der helixartigen Primärform überbrücken und welche an ihren beiden Längsenden an den Teilstegen (34) der helixförmigen Primärform jeweils zwischen zwei benachbarten Wendepunkten ansetzen,
**dadurch gekennzeichnet,**
**dass** die Umfangswandung (14) wenigstens eine an eine Stirnseite der Tragstruktur (24) angrenzende Endstruktur (22) aufweist die ein an seiner Stirnseite offenes Längsende der Umfangswandung (14) bildet und von einem (50') gebildet ist, der von Über den Umfang des Längsendes der Umfangswandung verteilt angeordneten,geschlossenen Zellen (54) gebildet ist, die in Umfangsrichtung über Umfangsverbindungsstege (56) paarweise miteinander verbunden sind.

2. Stent nach Anspruch 1, **dadurch** gekenzeichnet, dass die Teilstege (34) eine flache S-Form aufweisen, so dass sich eine flach S-förmige Tertiärform (36) des umlaufenden Stegs ergibt.

3. Stent nach Anspruch 1, **dadurch gekennzeichnet, dass** die Verbindungstege (40) eine flache S-Form (42) aufweisen.

4. Stent nach Anspruch 1, **dadurch** gekenzeichnet, dass die Verbindungstege (40) eine Haupterstreckungskomponente in Längsrichtung des Stentes haben.

5. Stent nach Anspruch 4, **dadurch** gekenzeichnet, dass die Verbingsstege im flachen Winkel zur Längsachse (12) des Stents (10) verlaufen.

6. Stent nach Anspruch 1, **dadurch** gekenzeichnet, dass jeweils eine Helixwindung von mehreren Verbindungsstegen überbrückt ist.

7. Stent nach Anspruch 6, **dadurch gekennzeichnet, dass** jeweils eine Helixwindung von 2 bis 5, über die Helixwindung in Umfangsrichtung im wesentlichen gleichmäßig verteilten Verbindungstegen (40) überbrückt ist.

8. Stent nach Anspruch 6 oder 7, **gekennzeichnet durch** eine Tragstruktur (24), welche mehrere Helixwindungen aufweist, welche jeweils **durch** Verbindungsstege (40) derart überbrückt sind, dass die Verbindungsstege benachbarter Helixwindungen in Umfangsrichtung (u) des Stents (10) zueinander versetzt sind.

9. Stent nach Anspruch 1, **dadurch gekennzeichnet, dass** die Wendeabschnitte (32) abgerundet ausgeführt sind.

## Claims

1. A stent
having a longitudinal axis (12) and
having a circumferential wall (14),
which is formed at least over a partial length of the stent (10) by a mesh-like supporting structure (24) open at two face sides, which comprises exactly one strut (16) wrapped helically about the longitudinal axis such that a helical primary shape (26) of the peripheral strut having more than one helical winding is obtained,
the strut (16), in addition the helical primary shape (26), having a zig-zag-shaped or meander-shaped secondary shape (30) comprising inflection points and related inflection sections (32), between which partial struts (34) of the peripheral strut having an extension component in the longitudinal direction of the stent run and
the supporting structure further comprising connecting struts (40), each of which bridges a helical winding of the helical primary shape and which, at the two longitudinal ends thereof, join the partial struts (34) of the helical primary shape between two adjoining inflection points in each case,
**characterized in that**
the circumferential wall (14) comprises at least one end structure (22), which adjoins a face side of the supporting structure (24) and which forms a longitudinal end of the circumferential wall (14) open at the face side thereof and is formed by a strut ring (50'), which is formed by closed cells (54) that are distributed over the circumference of the longitudinal end of the circumferential wall and connected to each other in pairs in the circumferential direction by way of circumferential connecting struts (56).

2. The stent according to claim 1, **characterized in that** the partial struts (34) have a flat S shape, whereby a flat S-shaped tertiary shape (36) of the peripheral strut is obtained.

3. The stent according to claim 1, **characterized in that** the connecting struts (40) have a flat S shape (42).

4. The stent according to claim 1, **characterized in that** the connecting struts (40) have a main extension component in the longitudinal direction of the stent.

5. The stent according to claim 4, **characterized in that** the connecting struts extend at a flat angle with respect to the longitudinal axis (12) of the stent (10).

6. The stent according to claim 1, **characterized in that** each helical winding is bridged by a plurality of connecting struts.

7. The stent according to claim 6, **characterized in that** each helical winding is bridged by 2 to 5 connecting struts (40), which are substantially uniformly distributed over the helical winding in the circumferential direction.

8. The stent according to claim 6 or 7, **characterized by** a supporting structure (24), which comprises a plurality of helical windings, each of which is bridged by connecting struts (4) such that the connecting struts of adjoining helical windings are offset from each other in the circumferential direction (u) of the stent (10).

9. The stent according to claim 1, **characterized in that** the inflection sections (32) are configured in a rounded manner.

## Revendications

1. Stent
- avec un axe longitudinal (12) et
- avec une paroi circonférentielle (14)
qui est formée au moins sur une longueur partielle du stent (10), par une structure porteuse (24) du genre grille, ouverte sur deux côtés avant et comprenant exactement une nervure (16) périphérique du genre hélice entourant l'axe longitudinal, de manière à donner à la nervure périphérique une forme primaire du genre hélice (26), avec plus d'une spire d'hélice,
dans lequel, en plus de la forme primaire (26) du genre hélice, la nervure (16) possède une forme secondaire (30) en forme de méandres ou du genre zigzag, avec des points d'inflexion et des sections d'inflexion (32) correspondantes, entre lesquelles s'étendent des nervures partielles (34) de la nervure périphérique, avec une composante d'extension dans le sens longitudinal du stent, et
dans lequel la structure porteuse comprend en outre des nervures de liaison (40), qui pontent chacune une spire d'hélice de la forme primaire du genre hélice et qui, à leurs deux extrémités longitudinales, rejoignent les nervures partielles (34) de la forme primaire du genre hélice, respectivement entre deux points d'inflexion voisins,
**caractérisé en ce que**
la paroi circonférentielle (14) comporte au moins une structure finale (22)adjacente à un côté avant de la structure porteuse (24), formant une extrémité longitudinale de paroi circonférentielle (14), ouverte du côté avant, et étant constituée d'un anneau de nervure (50'), formé par des cellules fermées (54), réparties sur le pourtour de l'extrémité longitudinale de la paroi circonférentielle, qui sont reliées entre elles par paires dans le sens du pourtour par des nervures de liaison périphériques (56).

2. Stent selon la revendication 1, **caractérisé en ce que** les nervures partielles (34) présentent une forme plate en S, de manière à donner à la nervure périphérique une forme tertiaire (36) plate en forme de S.

3. Stent selon la revendication 1, **caractérisé en ce que** les nervures de liaison (40) présentent une forme plate en S (42).

4. Stent selon la revendication 1, **caractérisé en ce que** les nervures de liaison (40) possèdent une composante de développement principale dans le sens longitudinal du stent.

5. Stent selon la revendication 4, **caractérisé en ce que** les nervures de liaison s'étendent en un angle plat par rapport à l'axe longitudinal (12) du stent.

6. Stent selon la revendication 1, **caractérisé en ce que** chacune des spires d'hélice est pontée par plusieurs nervures de liaison.

7. Stent selon la revendication 6, **caractérisé en ce que** chacune des spires d'hélice est pontée par 2 à 5 nervures de liaison (40) réparties de façon essentiellement régulière dans le sens du pourtour sur la spire d'hélice.

8. Stent selon la revendication 6 ou 7, **caractérisé par** une structure porteuse (24) comportant plusieurs spires d'hélice, qui sont chacune pontées de telle manière par des nervures de liaison (40), que les nervures de liaison des spires d'hélice voisines sont décalées les unes par rapport aux autres dans le sens du pourtour (u) du stent (10).

9. Stent selon la revendication 1, **caractérisé en ce que** les sections d'inflexion (32) sont conçues de manière arrondie.
